(19) Europäisches Patentamt
European Patent Office
Office européen des brevets

(11) Veröffentlichungsnummer: **0 047 719**
**B1**

(12) # EUROPÄISCHE PATENTSCHRIFT

(45) Veröffentlichungstag der Patentschrift: **28.11.84**

(51) Int. Cl.³: **C 02 F 3/34**, C 12 P 17/12// C12R1/38

(21) Anmeldenummer: **81810365.7**

(22) Anmeldetag: **04.09.81**

(54) **Verfahren zum Abbau von 2,4-Dihydroxy-6-amino-s-triazin-Derivaten.**

(30) Priorität: **10.09.80 CH 6796/80**

(43) Veröffentlichungstag der Anmeldung: **17.03.82 Patentblatt 82/11**

(45) Bekanntmachung des Hinweises auf die Patenterteilung: **28.11.84 Patentblatt 84/48**

(84) Benannte Vertragsstaaten: **CH DE FR GB IT LI**

(56) Entgegenhaltungen:
**AT-B- 224 572**
**DD-A- 109 607**

(73) Patentinhaber: **CIBA-GEIGY AG**
**Postfach**
**CH-4002 Basel (CH)**

(72) Erfinder: **Cook, Alasdair M., Dr.**
**Etzelstrasse 61**
**CH-8820 Wädenswil (CH)**
Erfinder: **Hütter, Ralf, Prof. Dr.**
**Rüttistrasse 72**
**CH-8044 Gockhausen (CH)**

**EP 0 047 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung bezieht sich auf ein Verfahren zum Abbau von 2,4-Dihydroxy-6-amino-s-triazin-Derivaten der Formel I

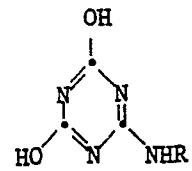

(I)

in welcher R einen aliphatischen oder cycloaliphatischen Rest mit 1 bis 6 Kohlenstoffatomen bedeutet, in Abwässern.

Bei der Herstellung von Triazinherbiziden, z. B. 2-Äthylamino-4-isopropylamino-6-chlor-s-triazin (Atrazin), 2,4-Bis-Äthylamino-6-chlor-s-triazin (Siamazin) oder 2,4-Bis-Isopropylamino-6-chlor-s-triazin (Propazin) durch Umsetzung von Cyanurchlorid mit entsprechenden Alkylaminen fällt Abwasser an, das ausser dem gewünschten herbiziden Wirkstoff auch verschiedene 6-Hydroxy-alkylamino-s-triazine enthält, wie z. B. 2-Amino-4-äthylamino-6-hydroxy-s-triazin (N-Äthylammelin), 2-Amino-4-isopropylamino-6-hydroxy-s-hydroxy-s-triazin (N-Isopropylammelin), 2,6-Bis-Hydroxy-4-äthylamino-s-triazin (N-Äthylammelid) oder 2,6-Bis-Hydroxy-4-isopropylamino-s-triazin (N-Isopropylammelid). Der Abbau solcher Verbindungen ist aus ökologischen Gründen wünschenswert.

Es ist bekannt, dass man Alkylamino-s-triazine oxidativ dealkylieren kann (J. Agr. Food Chem. *19* (3), 572-573, 1971). Die hierbei gebildeten Amino-s-triazine können durch Hydrolyse in saurem Medium weiter zu Cyanursäure umgewandelt werden, die hydrolytisch nicht weiter abbaubar ist.

Alkylamino-s-triazine der vorgenannten Art können auch direkt durch energische Hydrolyse in saurem Medium in Cyanursäure und Alkylamin gespalten werden (Journal of Chromatography *100*, 175-179, 1974). Ferner wurde bereits früher über den biologischen Abbau von 2,4-Bis-Alkylamino-6-chlor-s-triazinen zu Ammelid berichtet (J. Agr. Food Chem. *13*, 369-372, 1965). Der Abbau von Verunreinigungen, wie sie im Abwasser von Anlagen zur Herstellung von Triazinherbiziden vorhanden sind, führt also bei Anwendung der bisher bekannten, chemischen Methoden lediglich bis zur Cyanursäure. Nach dem vorgenannten biologischen Verfahren zum Abbau von 2,4-Bis-Alkylamino-6-chlor-s-triazinen wird dagegen Ammelid als Endprodukt des Abbaus erhalten, was eine anschliessende chemische Hydrolyse zur Cyanursäure notwendig macht.

Cyanursäure kann mikrobiologisch abgebaut werden (Arch. Microbiol. *67*, 1-5, 1969 ; J. Environm. Qual. *4*, 134-139, 1975 ; Appl. Microbiol. *28*, 1004-1008, 1974, DE-OS 25 21 842 und DE-OS 29 23 794), doch sind die Verfahren im allgemeinen langsam oder beschränken sich auf den Abbau der Cyanursäure, ohne dass dieselben Stämme auch Amino-s-triazin-derivate, welche an der Aminogruppe einen aliphatischen oder cycloaliphatischen Rest tragen, angreifen könnten.

Die vorgenannten Verfahren zum mikrobiellen Abbau von solchen Amino-s-triazin-Derivaten sind für praktische Zwecke nicht anwendbar, da die Abbauleistungen insgesamt zu gering sind. Ein weiterer Nachteil besteht darin, dass im allgemeinen keine vollständige Mineralisierung der Verbindungen stattfindet.

Es ist daher das Ziel der vorliegenden Erfindung, ein Verfahren zum Abbau von 2,4-Dihydroxy-6-amino-s-triazin-Derivaten der Formel I bereitzustellen, welches eine für praktische Zwecke ausreichend hohe Abbauleistung besitzt.

Es wurde nun gefunden, dass Stämme der Bakterien-Gattung *Pseudomonas*, nämlich *Pseudomonas sp.* NRRL B-12228 un *Pseudomonas sp.* NRRL B-12229 Verbindungen der Formel I als einzige Stickstoffquelle verwerten können.

Für den vollständigen Abbau von Verbindungen der Formel I zu Kohlendioxid und Ammoniak (in nicht-wachsenden Kulturen) oder zu Kohlendioxid und Zellmasse (in wachsenden Kulturen) eignet sich vor allem der Bakterienstamm *Pseudomonas sp.* NRRL B-12228.

Das erfindungsgemässe Verfahren zum Abbau von in Abwässern enthaltenen 2,4-Dihydroxy-6-amino-s-triazin-Derivaten der Formel I ist daher dadurch gekennzeichnet, dass man das Abwasser unter aeroben Bedingungen mit einem der Stämme *Pseudomonas sp.* NRRL B-12228 oder *Pseudomonas sp.* NRRL B-12229 in Kontakt bringt, wodurch die im Abwasser enthaltenen 2,4-Dihydroxy-6-amino-s-triazin-Derivate der Formel I schnell und vollständig abgebaut werden können.

Das erfindungsgemässe Verfahren eignet sich auch zum vollständigen Abbau von Triazinen in Abwässern, welche neben 2,4-Dihydroxy-6-amino-s-triazin-Derivaten der Formel I auch 2-Hydroxy-4,6-diamino-s-triazin-Derivate der Formel II,

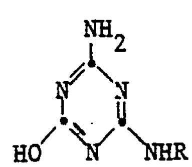

(II)

in welcher R für einen aliphatischen oder cycloaliphatischen Rest mit 1 bis 6 Kohlenstoffatomen steht, enthalten, wenn man das Abwasser vor oder während des Abbaus von 2,4-Dihydroxy-6-amino-s-triazin-Derivaten der Formel I zur Überführung von vorhandenen 2-Hydroxy-4,6-diamino-s-triazin-Derivaten der Formel II in 2,4-Dihydroxy-6-amino-s-triazin-Derivate der Formel I unter aeroben Bedingungen mit *Pseudomonas sp.* NRRL B-12227 in Kontakt bringt.

Besonders gute Resultate werden beim Abbau von 2,4-Dihydroxy-6-alkylamino-s-triazin-Derivaten der Formel Ia,

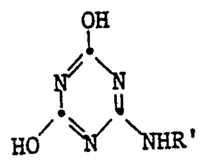

(Ia)

in welcher R' für einen Alkylrest mit 1 bis 6 Kohlenstoffatomen steht, sowie von 2-Hydroxy-4-amino-6-alkylamino-s-triazin-Derivaten der Formel IIa,

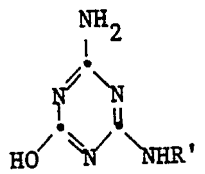

(IIa)

in welcher R' für einen Alkylrest mit 1 bis 6 Kohlenstoffatomen steht, erzielt, vor allem aber beim Abbau von Verbindungen der Formel Ib,

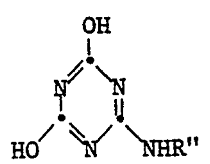

(Ib)

in welcher R'' für eine Äthyl- oder Isopropylgruppe steht, d. h. von 2,4-Dihydroxy-6-äthylamino-s-triazin (N-Äthylammelid) und 2,4-Dihydroxy-6-isopropylamino-s-triazin (N-Isopropylammelid) sowie von Verbindungen der Formel IIb,

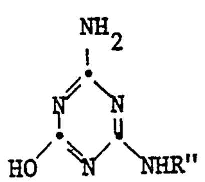

(IIb)

in welcher R'' für eine Äthyl- oder Isopropylgruppe steht, d. h. von 2-Hydroxy-4-amino-6-äthylamino-s-triazin (N-Äthylammelin) und 2-Hydroxy-4-amino-6-isopropylamino-s-triazin (N-Isopropylammelin).

Drei erfindungsgemäss zu verwendende Stämme von *Pseudomonas sp.* wurden am 18. Juli 1980 bei der Agricultural Research Culture Collection, Northern Regional Research Center (= NRRL), Peoria, Illinois 61604 — US, hinterlegt und unter den Ordnungsnummern B-12227, B-12228 und B-12229 registriert.

Der Stamm *Pseudomonas sp.* NRRL B-12227 wurde aus dem Klärschlamm der Abwasserreinigungsanlage Werdhölzli, Zürich (Schweiz), isoliert.

Die Stämme *Pseudomonas sp.* NRRL B-12228 und *Pseudomonas sp.* NRRL B-12229 wurden aus einer Mischung von Erdproben isoliert, die z. T. von den Versuchsfeldern der Ciba-Geigy AG in Bex und Vufflens (Schweiz), z. T. von Versuchsfeldern der Schweiz. Landwirtschaftlichen Forschungsstation Wädenswil (Schweiz) stammen, und die eine bis sieben Behandlungen mit s-Triazinherbiziden erhalten hatten.

Die drei Stämme *Pseudomonas sp.* NRRL B-12227, *Pseudomonas sp.* NRRL B-12228 und *Pseudomonas sp.* NRRL B-12229 sind als *Pseudomonas*-Stämme identifiziert worden, da es sich um strikt aerobe, oxidate-positive und bewegliche Stäbchen (ca. 0.5 bis 1.5 × 1 bis 3 μm) handelt, die ohne Vitamine im pH-Bereich 6 bis 8 wachsen und sowohl Glukose wie auch Azetat als C-Quelle verwerten können. Elektronenmikroskopische Beobachtungen zeigen ferner eine für gram-negative Bakterien typische Zellwandstruktur, sowie eine polare, multitriche Begeisselung. Die Organismen können keiner der in Stanier et al. (Stanier, R.Y., N.J. Palleroni & M. Doudoroff ; J. Gen. Microbiol. *43,* 159-271, 1966) beschriebenen Spezies zugeordnet werden. Die Stämme *Pseudomonas sp.* NRRL B-12228 und *Pseudomonas sp.* NRRL B-12229 sind sich sehr ähnlich, weshalb bei der detaillierten Beschreibung nur der Stamm *Pseudomonas sp.* NRRL B-12228 aufgeführt wird.

Auf Nähragar-Platten wachsen die Stämme bei 30 °C innerhalb von zwei Tagen zu 1 mm (*Pseudomonas sp.* NRRL B-12228) bzw. 2 mm (*Pseudomonas sp.* NRRL B-12227) grossen, weisslich-beigefarbenen, matten (*Pseudomonas sp.* NRRL B-12227) oder schleimig-glänzenden (*Pseudomonas sp.* NRRL B-12228) Kolonien mit glattem Rand aus. Das Wachstum ist gut im Bereich von 24 bis 37 °C, während beide Stämme unterhalb 20 °C nur noch langsam und ab 41 °C und darüber gar nicht mehr wachsen.

Nachfolgend sind weitere Merkmale der Stämme *Pseudomonas sp.* NRRL B-12227 und *Pseudomonas sp.* NRRL B-12228 aufgeführt :

| Merkmal | *Pseudomonas sp.* NRRL B-12227 | *Pseudomonas sp.* NRRL B-12228 |
|---|---|---|
| Zahl der Geisseln | > 1 | > 1 |
| Fluoreszierendes Pigment | 0 | 0 |
| Phenazin-Pigment | 0 | 0 |
| Methionin-Bedürfnis | 0 | 0 |
| Denitrifikation | 0 | 0 |
| Wachstum bei 4 °C | 0 | 0 |
| Wachstum bei 41 °C | 0 | 0 |
| Stärke-Hydrolyse | + | + |
| Oxidase-Reaktion | + | + |
| Arginin-dihydrolase-Reaktion | 0 | 0 |
| Verwertung von Kohlenstoffquellen : | | |
| D-Glucose | + | + |
| Trehalose | 0 | + |
| Cellobiose | 0 | 0 |
| Maltose | + | + |
| Stärke | + | + |
| Inositol | 0 | 0 |
| 2-Ketogluconat | 0 | + |
| Maleat | 0 | + |
| Glykolat | 0 | + |
| DL-Lactat | + | + |
| Adipat | + | + |
| Testosteron | 0 | 0 |
| Acetamid | 0 | 0 |
| D-Tryptophan | + | + |
| p-Hydroxybenzoat | + | + |
| Glycerin | + | + |
| Succinat | + | + |
| Acetat | + | + |
| Wachstum auf McConkey-Agar | 0 | + |

Die erfindungsgemäss zu verwendenden Bakterien der Gattung *Pseudomonas* werden zunächst auf einem geeigneten Basismedium gezüchtet und anschliessend wässrigen Lösungen der abzubauenden Substrate zugesetzt oder sie werden direkt auf Medien, welche die abzubauenden Substrate enthalten, gezüchtet.

Ein geeignetes Basismedium ist beispielsweise wie folgt zusammengesetzt :

| | |
|---|---|
| Kaliumphosphat-Puffer, pH 7.3 | 10 mM |
| Magnesiumsulfat-Heptahydrat | 0.25 mM |
| Spurenelementlösung nach Pfennig und Lippert (Arch. Microbiol *55,* 245, 1966), ergänzt mit 100 mg pro Liter Calciumchlorid-Dihydrat | 5 ml/Liter |
| Milchsäure | 10 mM |
| Stickstoffquelle | 2.5 mM Stickstoff |

Als Stickstoffquellen können Ammoniak, Cyanursäure, Ammelid, Ammelin oder Verbindungen der

Formel I (für *Pseudomonas sp.* NRRL B-12228 und *Pseudomonas sp.* NRRL B-12229) oder Verbindungen der Formel II (*für Pseudomonas sp.* NRRL B-12227) verwendet werden.

Das Verfahren zum Abbau von Verbindungen der Formel I kann beispielsweise so durchgeführt werden, dass man ein wässriges Medium, in welchem die Verbindungen in gelöster Form zusammen mit geeigneten Salzen und einer geeigneten Kohlenstoffquelle vorliegen, mit Kulturen des Stammes *Pseudomonas sp.* NRRL B-12228 animpft. Als geeignete Salze sind beispielsweise die im vorstehend beschriebenen Basismedium angegebenen anzusehen. Geeignete Kohlenstoffquellen sind z. B. Glucose, Acetat oder Milchsäure. Als besonders günstig erweist sich die Durchführung des Verfahrens mit wachsenden Zellen von *Pseudomonas sp.* NRRL B-12228.

Das Verfahren kann in entsprechender Weise unter Animpfen mit *Pseudomonas sp.* NRRL B-12227 durchgeführt werden, um Verbindungen der Formel II in Verbindungen der Formel I zu überführen.

Man kann auch separat vorgezüchtete Kulturen von *Pseudomonas sp.* NRRL B-12228 und *Pseudomonas sp.* NRRL B-12227 in einem Verhältnis von 1 : 1 (Zellmasse von *Pseudomonas sp.* NRRL B-12228 : Zellmasse von *Pseudomonas sp.* NRRL B-12227) vermischen, anschliessend in 10 mM Phosphatpuffer, pH 7.3, inkubieren und mit dem wässrigen Medium in Kontakt bringen.

Anstelle von *Pseudomonas sp.* NRRL B-12228 lässt sich auch *Pseudomonas sp.* NRRL B-12229 verwenden, jedoch ist der Stamm *Pseudomonas sp.* NRRL B-12228 eindeutig zu bevorzugen.

Das erfindungsgemässe Verfahren wird durch die nachfolgenden Beispiele näher erläutert.

## Beispiel 1

Abbau von N-Äthylammelid zu Kohlendioxid und Zellmasse durch wachsende Kulturen von *Pseudomonas sp.* NRRL B-12228

Von einer Schrägagarkultur auf Basis-Agar, welcher dem nachfolgend angegebenen Basis-Medium mit Zusatz von 2 % Agar (Difco Noble Agar) entspricht, wird eine Probe des Stammes *Pseudomonas sp.* NRRL B-12228 entnommen und in Basis-Medium folgender Zusammensetzung geimpft :

| | |
|---|---|
| Kaliumphosphat-Puffer, pH 7.3 | 10 mM |
| Magnesiumsulfat-Heptahydrat | 0.25 mM |
| Spurenelementlösung nach Pfennig und Lippert (Arch. Microbiol. *55*, 245, 1966), ergänzt mit 100 mg pro Liter Calciumchlorid-Dihydrat | 5 ml/Liter |
| Milchsäure | 10 mM |
| N-Äthylammelid | 0.6 mM |

Die Suspension wird auf einer rotierenden Schüttelmaschine (180 rpm) bei 30 °C inkubiert. In Abständen von ca. 1 Stunde werden Proben entnommen, in denen die relative Bakterienmenge durch Messung der optischen Dichte bei 546 nm ($OD_{546}$) und die Menge des verbleibenden N-Äthyl-ammelins sowie des entstandenen N-Äthyl-ammelids durch Hochdruckflüssigkeitschromatographie (HPLC) verfolgt werden.

Die Versuchsergebnisse sind in Tabelle 1 dargestellt.

Tabelle 1

| Stickstoffquelle im Medium (Züchtungsbeginn, je 2.5 mM N) | (Züchtungsende) | Wachstumsertrag (g Protein/Mol verwerteter N) | Wachstumsrate $\mu(h^{-1})$ | spez. Abbaurate (mkat/kg *) Protein) |
|---|---|---|---|---|
| Ammoniak | nicht mehr nachweisbar | 43 | 0.29 | 1.9 |
| N-Äthylammelid | | 38 | — | — |

— = nicht bestimmt
*) kat = Katal (= mol/sec)

Aus den angegebenen Daten ist ersichtlich, dass der Stamm *Pseudomonas sp.* NRRL B-12228 den im N-Äthylammelid vorhandenen Stickstoff vollständig in seine Zellmasse einzubauen vermag.

## Beispiel 2

Abbau von N-Isopropylammelid zu Kohlendioxid und Zellmasse durch wachsende Kulturen von *Pseudomonas sp.* NRRL B-12228

Unter Anwendung der im Beispiel 1 angegebenen Versuchsanordnung wird das Verfahren mit N-Isopropylammelid als Stickstoffquelle anstelle von N-Äthylammelid ausgeführt.

Die Versuchsergebnisse sind in Tabelle 2 dargestellt.

Tabelle 2

| Stickstoffquelle im Medium (Züchtungsbeginn, je 2.5 mM N) | (Züchtungsende) | Wachstumsertrag (g Protein/Mol verwerteter N) | Wachstumsrate $\mu(h^{-1})$ | spez. Abbaurate (mkat/kg Protein) |
|---|---|---|---|---|
| Ammoniak | nicht mehr | 43 | 0.29 | 1.9 |
| N-Isopropylammelid | nachweisbar | 44 | 0.28 | 0.44 |

Aus den angegebenen Daten ist ersichtlich, dass der Stamm *Pseudomonas sp.* NRRL B-12228 den im N-Isopropylammelid vorhandenen Stickstoff vollständig in seine Zellmasse einzubauen vermag.

Beispiel 3

Deaminierung von N-Äthylammelin zu N-Äthylammelid mit *Pseudomonas sp.* NRRL B-12227

Von einer Schrägagarkultur auf Basis-Agar, welcher dem nachfolgend angegebenen Basis-Medium mit Zusatz von 2 % Agar (Difco Noble Agar) entspricht, wird eine Probe des Stammes *Pseudomonas sp.* NRRL B-12227 entnommen und in Basis-Medium folgender Zusammensetzung geimpft :

| | |
|---|---|
| Kaliumphosphat-Puffer, pH 7.3 | 10 mM |
| Magnesiumsulfat-Heptahydrat | 0.25 mM |
| Spurenelementlösung nach Pfennig und Lippert (Arch. Microbiol *55*, 245, 1966), ergänzt mit 100 mg pro Liter Calciumchlorid-Dihydrat | 5 ml/Liter |
| Milchsäure | 10 mM |
| N-Äthylammelin | 0.5 mM |

Die Suspension wird auf einer rotierenden Schüttelmaschine (180 rpm) bei 30 °C inkubiert. In Abständen von ca. 1 Stunde werden Proben entnommen, in denen die relative Bakterienmenge durch Messung der optischen Dichte bei 546 nm ($OD_{546}$) und die Menge des verbleibenden N-Äthyl-ammelins sowie des entstandenen N-Äthyl-ammelids durch Hochdruckflüssigkeitschromatographie (HPLC) verfolgt werden.

Die Versuchsergebnisse sind in Tabelle 3 dargestellt.

Tabelle 3

| Stickstoffquelle im Medium (Züchtungsbeginn) | (Züchtungsende) | Wachstumsertrag (g Protein/Mol verwerteter N) | Wachstumsrate $\mu(h^{-1})$ | Umsatzrate (mkat/kg) Protein) |
|---|---|---|---|---|
| N-Äthylammelin | N-Äthylammelid | 60 | 0.13 | 0.6 |

Beispiel 4

Deaminierung von N-Isopropylammelin zu N-Isopropylammelid mit *Pseudomonas sp.* NRRL B-12227

Unter Anwendung der im Beispiel 3 angegebenen Versuchsanordnung wird das Verfahren mit N-Isopropylammelin als tickstoffquelle anstelle von N-Äthylammelin ausgeführt.

Die Versuchsergebnisse sind in Tabelle 4 dargestellt.

Tabelle 4

| Stickstoffquelle im Medium (Züchtungsbeginn) | (Züchtungsende) | Wachstumsertrag (g Protein/Mol verwerteter N) | Wachstumsrate $\mu(h^{-1})$ | Umsatzrate (mkat/kg) Protein) |
|---|---|---|---|---|
| N-Isopropylammelin | N-Isopropylammelid | 60 | 0.12 | 0.5 |

Beispiel 5

Mineralisierung von N-Äthylammelin zu Kohlendioxid und Ammoniak durch eine Mischung von ruhenden Zellen von *Pseudomonas sp.* NRRL B-12228 und *Pseudomonas sp.* NRRL B-12227

Die Stämme *Pseudomonas sp.* NRRL B-12228 und *Pseudomonas sp.* NRRL B-12227 werden gemäss den in den Beispielen 1 und 3 dargestellten Bedingungen gezüchtet. Nach Abschluss des Wachstums der Einzelstämme werden aliquote Mengen der beiden Stämme gemischt, und es wird eine Suspension von 0,3 mg Protein/ml in 10 mM Kaliumphosphatpuffer, pH 7.3, hergestellt. Diesem Puffer werden 0.5 mM N-Äthylammelin zugegeben, und die Entstehung von Kohlendioxid und Ammoniak wird verfolgt, ebenso wie das Verschwinden des Substrats.

Die Ergebnisse sind in Tabelle 5 dargestellt (die Versuchsdauer Betrug 5 Stunden).

Tabelle 5

| Organismen (Stammischung) | Substratmenge (Versuchsbeginn | (Versuchsende) | Ammoniak (entstanden bis Versuchsende) | Kohlendioxid (entstanden bis Versuchsende) |
|---|---|---|---|---|
| A (0.15 mg Protein/ml) und D (0.15 mg Protein/ml) | 0.5 mM N-Äthylammelin | nicht mehr nachweisbar | 2.3 mM | 83 %[1)] |

A : *Pseudomonas sp.* NRRL B-12227 D : *Pseudomonas sp.* NRRL B-12228

[1]) Entspricht Prozent der Eingabe. Auch bei Verwendung von Bicarbonat beträgt die Kohlendioxidausbeute lediglich 85 % ; damit entspricht die in der Tabelle angegebene Ausbeute nahezu 100 %. Der Versuch wird mit $^{14}C$-markiertem Material (Triazin ringmarkiert) durchgeführt.

Die vorstehenden Daten zeigen deutlich, dass die Mischung der Stämme *Pseudomonas sp.* NRRL B-12227 und *Pseudomonas sp.* NRRL B-12228 N-Äthylammelin effizient zu Kohlendioxid und Ammoniak abbauen kann.

Beispiel 6

Mineralisierung von N-Isopropylammelin zu Kohlendioxid und Ammoniak durch eine Mischung von ruhenden Zellen von *Pseudomonas sp.* NRRL B-12228 und *Pseudomonas sp.* NRRL B-12227

Unter Anwendung der im Beispiel 5 angegebenen Versuchsanordnung wird das Verfahren mit N-Isopropylammelin als Stickstoffquelle anstelle von N-Äthylammelin ausgeführt.

Die Versuchsergebnisse sind in Tabelle 6 dargestellt (die Versuchsdauer betrug 5 Stunden).

(Siehe Tabelle 6 Seite 8 f.)

Tabelle 6

| Organismen (Stammischung) | Substratmenge (Versuchsbeginn) | Substratmenge (Versuchsende) | Ammoniak (entstanden bis Versuchsende) | Kohlendioxid (entstanden bis Versuchsende) 1) |
|---|---|---|---|---|
| A (0.15 mg Protein/ml) und D (0.15 mg Protein/ml) | 0.5 mM N-Isopropylammelin | nicht mehr nachweisbar | 2.5 mM | 84 % 1) |

A : *Pseudomonas sp.* NRRL B-12227 D : *Pseudomonas sp.* NRRL B-12228

1) Entspricht Prozent der Eingabe. Auch bei Verwendung von Bicarbonat beträgt die Kohlendioxidausbeute lediglich 85 % ; damit entspricht die in der Tabelle angegebene Ausbeute nahezu 100 %. Der Versuch wird mit $^{14}C$-markiertem Material (Triazin ringmarkiert) durchgeführt.

Die vorstehenden Daten zeigen deutlich, dass die Mischung der Stämme *Pseudomonas sp.* NRRL B-12227 und *Pseudomonas sp.* NRRL B-12228 N-Isopropylammelin effizient zu Kohlendioxid und Ammoniak abbauen kann.

Die vorliegende Erfindung umfasst neben den vorstehend genannten Stämmen von *Pseudomonas* auch die Verwendung dieser Bakterien oder Mutanten im Rahmen der vorliegenden Erfindung.

**Ansprüche**

1. Verfahren zum Abbau von 2,4-Dihydroxy-6-amino-s-triazin-Derivaten der Formel I,

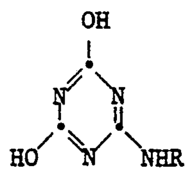

(I)

in welcher R einen aliphatischen oder cycloaliphatischen Rest mit 1 bis 6 Kohlenstoffatomen bedeutet, in Abwässern, dadurch gekennzeichnet, dass man das Abwasser unter aeroben Bedingungen mit einem der Stämme *Pseudomonas sp.* NRRL B-12228 oder *Pseudomonas sp.* NRRL B-12229 oder von zum Abbau von Verbindungen der Formel I geeigneten Mutanten dieser Stämme in Kontakt bringt.

2. Verfahren nach Anspruch 1, dadurch gekennzeichnet, dass man Abwasser, welches eine Verbindung der Formel Ia,

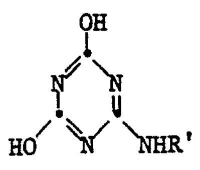

(Ia)

in welcher R' für einen Alkylrest mit 1 bis 6 Kohlenstoffatomen steht, enthält, mit einem der *Pseudomonas*-Stämme in Kontakt bringt.

3. Verfahren nach Anspruch 2, dadurch gekennzeichnet, dass man Abwasser, welches eine Verbindung der Formel Ib,

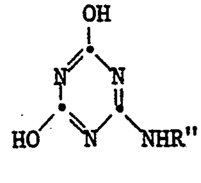

(Ib)

in welcher R" für eine Äthyl- oder Isopropylgruppe steht, enthält, mit einem der *Pseudomonas*-Stämme in Kontakt bringt.

4. Verfahren nach Anspruch 1, dadurch gekennzeichnet, dass man das Verfahren mit *Pseudomonas sp.* NRRL B-12228 durchführt.

5. Verfahren nach Anspruch 4, dadurch gekennzeichnet, dass man das Verfahren mit wachsenden Zellen von *Pseudomonas sp.* NRRL B-12228 durchführt.

6. Verfahren nach Anspruch 1, dadurch gekennzeichnet, dass man das Abwasser vor oder während des Abbaus von 2,4-Dihydroxy-6-amino-s-triazin-Derivaten der Formel I zur Überführung von vorhandenen 2-Hydroxy-4,6-diamino-s-triazin-Derivaten der Formel II,

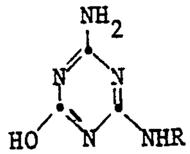

(II)

in welcher R einen aliphatischen oder cycloaliphatischen Rest mit 1 bis 6 Kohlenstoffatomen darstellt, in 2,4-Dihydroxy-6-amino-s-triazin-Derivate der Formel I unter aeroben Bedingungen mit *Pseudomonas sp.* NRRL B-12227 oder mit zur Überführung von Verbindungen der Formel II geeigneten Mutanten dieses Stammes in Kontakt bringt.

7. Verfahren nach Anspruch 2, dadurch gekennzeichnet, dass man das Abwasser vor oder während des Abbaus von 2,4-Dihydroxy-6-amino-s-triazin-Derivaten der Formel Ia zur Überführung von vorhandenen 2-Hydroxy-4,6-diamino-s-triazin-Derivaten der Formel IIa,

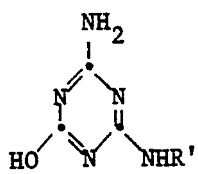

(IIa)

in welcher R' für einen Alkylrest mit 1 bis 6 Kohlenstoffatomen steht, in 2,4-Dihydroxy-6-amino-s-triazin-Derivate der Formel Ia unter aeroben Bedingungen mit *Pseudomonas sp.* NRRL B-12227 oder mit zur Überführung von Verbindungen der Formel IIa geeigneten Mutanten dieses Stammes in Kontakt bringt.

8. Verfahren nach Anspruch 3, dadurch gekennzeichnet, dass man das Abwasser vor oder während des Abbaus von 2,4-Dihydroxy-6-amino-s-triazin-Derivaten der Formel Ib zur Überführung von vorhandenen 2-Hydroxy-4,6-diamino-s-triazin-Derivaten der Formel IIb,

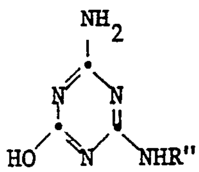

(IIb)

in welcher R" für eine Äthyl- oder Isopropylgruppe steht, in 2,4-Dihydroxy-6-amino-s-triazin-Derivate der Formel Ib unter aeroben Bedingungen mit *Pseudomonas sp.* NRRL B-12227 oder mit zur Überführung von Verbindungen der Formel IIb geeigneten Mutanten dieses Stammes in Kontakt bringt.

9. Bakterien des Stammes *Pseudomonas sp.* NRRL B-12228.

10. Bakterien des Stammes *Pseudomonas sp.* NRRL B-12229.

11. Bakterien des Stammes *Pseudomonas sp.* NRRL B-12227.

**Claims**

1. A process for decomposing 2,4-dihydroxy-6-amino-s-triazine derivatives of the formula I

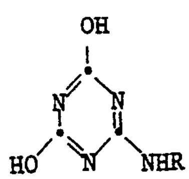

(I),

wherein R is an aliphatic or cycloaliphatic radical having 1 to 6 carbon atoms, in effluents, which process

comprises bringing the effluent, under aerobic conditions, into contact with either of the strains *Pseudomonas sp.* NRRL B-12228 or *Pseudomonas sp.* NRRL B-12229, or with mutants of these strains, which mutants are suitable for decomposing compounds of the formula I.

2. A process according to Claim 1, wherein effluent which contains a compound of the formula Ia

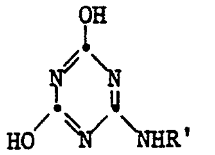

(Ia)

wherein R' is an alkyl group having 1 to 6 carbon atoms, is brought into contact with one of the *Pseudomonas* strains.

3. A process according to Claim 2, wherein effluent which contains a compound of the formula Ib

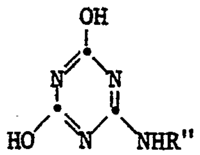

(Ib)

wherein R" is an ethyl or isopropyl group, is brought into contact with one of the *Pseudomonas* strains.

4. A process according to Claim 1, which process is performed with *Pseudomonas sp.* NRRL B-12228.

5. A process according to Claim 4, which process is performed with growing cells of *Pseudomonas sp.* NRRL B-12228.

6. A process according to Claim 1, wherein the effluent, before or during the decomposition of 2,4-dihydroxy-6-amino-s-triazine derivatives of the formula I, is brought into contact, for conversion of 2-hydroxy-4,6-diamino-s-triazine derivatives of the formula II present

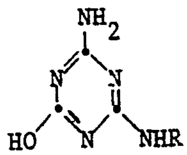

(II),

wherein R is an aliphatic or cycloaliphatic radical having 1 to 6 carbon atoms, into 2,4-dihydroxy-6-amino-s-triazine derivatives of the formula I, under aerobic conditions with *Pseudomonas sp.* NRRL B-12227, or with mutants of this strain which are suitable for the conversion of compounds of the formula II.

7. A process according to Claim 2, wherein the effluent, before or during the decomposition of 2,4-dihydroxy-6-amino-s-triazine derivatives of the formula Ia, is brought into contact, for conversion of 2-hydroxy-4,6-diamino-s-triazine derivatives of the formula IIa present

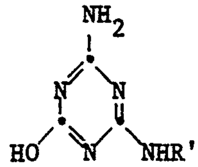

(IIa)

wherein R' is an alkyl group having 1 to 6 carbon atoms, into 2,4-dihydroxy-6-amino-s-triazine derivatives of the formula Ia, under aerobic conditions with *Pseudomonas sp.* NRRL B-12227, or with mutants of this strain which are suitable for the conversion of compounds of the formula IIa.

8. A process according to Claim 3, wherein the effluent, before or during the decomposition of 2,4-dihydroxy-6-amino-s-triazine derivatives of the formula Ib, is brought into contact, for conversion of 2-hydroxy-4,6-diamino-s-triazine derivatives of the formula IIb present

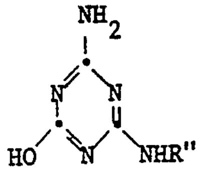

(IIb)

wherein R″ is an ethyl or isopropyl group, into 2,4-dihydroxy-6-amino-s-triazine derivatives of the formula Ib, unter aerobic conditions with *Pseudomonas sp.* NRRL B-12227, or with mutants of this strain which are suitable for the conversion of compounds of the formula IIb.

9. Bacteria of the strain *Pseudomonas sp.* NRRL B-12228.

10. Bacteria of the strain *Pseudomonas sp.* NRRL B-12229.

11. Bacteria of the strain *Pseudomonas sp.* NRRL B-12227.

**Revendications**

1. Procédé de dégradation de dérivés de 2,4-dihydroxy-6-amino-s-triazine de formule I

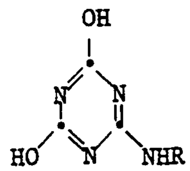

(I)

où R représente un radical aliphatique ou cycloaliphatique en $C_1$ à $C_6$, dans des eaux résiduaires, caractérisé en ce qu'on met en contact les eaux résiduaires dans des conditions aérobies avec l'une des souches *Pseudomonas sp.* NRRL B-12228 ou *Pseudomonas sp.* NRRL B-12229 ou des mutants de ces souches appropriés à la dégradation des composés de formule I.

2. Procédé selon la revendication 1, caractérisé en qu'on met en contact des eaux résiduaires contenant un composé de formule Ia

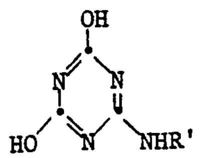

(Ia)

où R′ représente un radical alcoyle en $C_1$ à $C_6$, avec l'une des souches de *Pseudomonas.*

3. Procédé selon la revendication 2, caractérisé en ce qu'on met en contact un composé de formule Ib

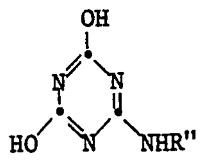

(Ib)

où R″ représente un groupe éthyle ou isopropyle, avec l'une des souches de *Pseudomonas.*

4. Procédé selon la revendication 1, caractérisé en ce qu'on conduit le procédé avec *Pseudomonas sp.* NRRL B-12228.

5. Procédé selon la revendication 4, caractérisé en ce qu'on conduit le procédé avec des cellules en croissance de *Pseudomonas sp.* NRRL B-12228.

6. Procédé selon la revendication 1, caractérisé en ce qu'on met en contact les eaux résiduaires, avant ou après la dégradation des dérivés de 2,4-dihydroxy-6-amino-s-triazine de formule I pour transformer les dérivés de 2-hydroxy-4,6-diamino-s-triazine présents de formule II,

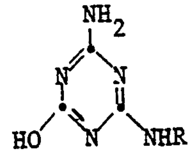

(II)

où R représente un radical aliphatique ou cycloaliphatique en $C_1$ à $C_6$, en dérivés de 2,4-dihydroxy-6-amino-s-triazine de formule I, dans des conditions aérobies, avec *Pseudomonas sp.* NRRL B-12227 ou avec des mutants de cette souche appropriés à la transformation de composés de formule II.

7. Procédé selon la revendication 2, caractérisé en ce qu'on met en contact les eaux résiduaires, avant ou après la dégradation des dérivés de 2,4-dihydroxy-6-amino-s-triazine de formule Ia pour transformer les dérivés de 2-hydroxy-4,6-diamino-s-triazine présents de formule IIa

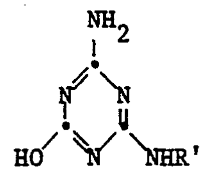

(IIa)

où R' représente un radical alcoyle en $C_1$ à $C_6$, en dérivés de 2,4 dihydroxy-6-amino-s-triazine de formule Ia, dans des conditions aérobies, avec *Pseudomonas sp.* NRRL B-12227 ou avec des mutants de cette souche appropriés à la transformation de composés de formule IIa.

8. Procédé selon la revendication 3, caractérisé en ce qu'on met en contact les eaux résiduaires, avant ou après la dégradation des dérivés de 2,4-dihydroxy-6-amino-s-triazine de formule Ib pour transformer les dérivés de 2-hydroxy-4,6-diamino-s-triazine présents de formule IIb

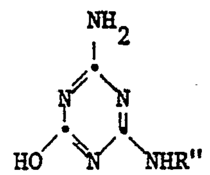

(IIb)

où R" représente un groupe éthyle ou isopropyle, en dérivés de 2,4-dihydroxy-6-amino-s-triazine de formule Ib, dans des conditions aérobies, avec *Pseudomonas sp.* NRRL B-12227 ou avec des mutants de cette souche appropriés à la transformation des composés de formule IIb.

9. Bactéries de souche *Pseudomonas sp.* NRRL B-12228.

10. Bactéries de souche *Pseudomonas sp.* NRRL B-12229.

11. Bactéries de souche *Pseudomonas sp.* NRRL B-12227.